Europäisches Patentamt

European Patent Office

Office européen des brevets

(11) Veröffentlichungsnummer: **0 300 201 B1**

(19)

## (12) EUROPÄISCHE PATENTSCHRIFT

(45) Veröffentlichungstag der Patentschrift: **22.01.92**

(21) Anmeldenummer: **88109709.1**

(22) Anmeldetag: **17.06.88**

(51) Int. Cl.⁵: **A61M 1/16**, A61M 1/34

---

(54) **Vorrichtung zur Hämodialyse und Hämofiltration.**

---

(30) Priorität: **23.06.87 DE 3720665**

(43) Veröffentlichungstag der Anmeldung:
**25.01.89 Patentblatt 89/04**

(45) Bekanntmachung des Hinweises auf die
Patenterteilung:
**22.01.92 Patentblatt 92/04**

(84) Benannte Vertragsstaaten:
**ES FR GB IT SE**

(56) Entgegenhaltungen:
**DD-A- 244 816**
**DE-A- 2 607 022**
**DE-A- 3 313 421**
**US-A- 4 370 983**

(73) Patentinhaber: **Schäl, Wilfried, Dr.-Ing.**
**Tannenwaldweg 27**
**W-6380 Bad Homburg(DE)**

(72) Erfinder: **Schäl, Wilfried, Dr.-Ing.**
**Tannenwaldweg 27**
**W-6380 Bad Homburg(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

Rank Xerox (UK) Business Services

EP 0 300 201 B1

**Beschreibung**

Die Erfindung betrifft eine Vorrichtung zur Hämodialyse und Hämofiltration, die dazu dient, das Blut eines Patienten in einem künstlich geschaffenen extrakorporalen Blutkreislauf durch Dialyse und/oder Filtration, ggf. kombiniert mit einer Zufuhr von Infusionslösungen in den Blutkreislauf, so zu behandeln, daß eine möglichst weitgehende Normalisierung der Zusammensetzung des Blutplasmas erreicht wird. Vorrichtungen dieser Art sind geeignet, die Funktion der Niere zu ersetzen und sind auch unter der Bezeichnung "künstliche Niere" bekannt.

Für den medizinischen Erfolg und für die Verträglichkeit einer solchen Behandlung ist es bekanntlich von großer Wichtigkeit, den Netto-Entzug von Flüssigkeit aus dem Blut genau zu erfassen und durch entsprechende Einstellung der Betriebsparameter von Hand oder automatisch zu regeln. Für diesen Zweck sind verschiedene Systeme bekannt.

Ausgehend von der Überlegung, daß der Entzug eines bestimmten Flüssigkeitsvolumens pro Zeiteinheit zwangsläufig mit einer entsprechenden Verminderung des zum Patienten pro Zeiteinheit zurückgeführten Blutvolumens verknüpft ist, wird in der Offenlegungsschrift DE 33 13 421 A1 z.B. vorgeschlagen, bei einer Dialysevorrichtung den extrakorporalen Blutkreislauf mit einem Differenzdurchflußmesser auszustatten und die so gewonnene Information in an sich bekannter Weise zur Steuerung des Flüssigkeitsentzuges zu benutzen.

Das hauptsächliche Problem bei der Realisierung einer Messung des Flüssigkeitsentzuges pro Zeiteinheit besteht jedoch darin, für die Messung des Blutflusses geeignete Durchflußmesser zur Verfügung zu haben.

In DE 33 13 421 A1 wurde hierzu vorgeschlagen, vorzugsweise elektromagnetische Durchflußmesser - vermutlich sind induktive Durchflußmesser gemeint- zu benutzen, die speziell ausgesucht Schläuche mit jeweils zwei eingesetzten Elektroden aufweisen sollen, an die ein elektromagnetisches Feld angelegt werden soll, um an ihnen eine der Differenz der Durchflußgeschwindigkeiten proportionale Spannung zu entnehmen. Abgesehen von dieser fehlerhaften Auffassung des Funktionsprinzips bleibt die Frage ungelöst, wie die technischen Schwierigkeiten, die der Erzielung einer für den vorgesehenen Zweck ausreichenden Genauigkeit und Betriebssicherheit entgegenstehen, gelöst werden könnten.

Zur Blutflußmessung nach dem Induktionsprinzip ist es notwendig, den Strömungskanal in ein quer zur Stromung gerichtetes, möglichst homogenes Magnetfeld zu bringen, so daß quer zur Strömungsrichtung und quer zur Richtung des Magnetfeldes mittels gegenüberliegend am Stömungskanal angeordneter Elektroden eine der magnetischen Flußdichte B, dem Elektrodenabstand d und der mittleren Strömungsgeschwindigkeit v proportionale induzierte Spannung U abgegriffen werden kann: $U = B^*d^*v$. Bei einem kreisförmigen Strömungsquerschnitt, wie er bei den Blutschläuchen vorliegt, sind außerdem die mittlere Strömungsgeschwindigkeit v, der Blutfluß Q (Volumen pro Zeiteinheit) und der Schlauch-Innendurchmesser di durch die Beziehung $v = 4^*Q/di^{2*}\pi$ miteinander verknüpft. Aus beiden Beziehungen resultiert $U = 4^*B^*Q^*d/di^{2*}\pi$, d.h. zur Erlangung einer ausreichenden Genauigkeit genügt es nicht, die beiden Schlauchdurchmesser di anzugleichen, sondern es müssen auch die Werte der magnetischen Flußdichte B und des Elektrodenabstandes d im Rahmen der geforderten Genauigkeit in Übereinstimmung gebracht werden.

Eine weitere Schwierigkeit beim Einsatz induktiver Durchflußmesser für den genannten Zweck ergibt sich daraus, daß die Differenz der induzierten Spannungen außerordentlich gering ist, so daß bei deren Verarbeitung durch die unvermeidlichen, um mehrere Größenordnungen höheren Störspannungen zusätzliche Fehler auftreten. Die Größenordnung der induzierten Spannungen ergibt sich aus folgender Abschätzung: Der Luftspalt des Magnetsystems muß mindestens dem Schlauch-Außendurchmesser entsprechen. Dieser beträgt typischerweise etwa 6 mm. Zur Erlangung eines annähernd homogenen Magnetfeldes im Bereich der Meßstelle muß die Querschnittsfläche des Luftspaltes um ein mehrfaches größer sein als das Quadrat des Schlauchdurchmessers, beispielsweise 10mm*10mm = 100 mm. Nach üblichen Dimensionierungsregeln für Elektromagnete kann in einem solchen Luftspalt mit einem Elektromagneten von noch akzeptabler Größe und Leistungsaufnahme (Gewicht einige 100 g, Leistung einige Watt) eine magnetische Flußdichte in der Größenordnung von 0,5 mT erzeugt werden. Für den Blutfluß gilt ein typischer Wert von Q = 200 ml/min. Unter Berücksichtigung eines Schlauchinnendurchmessers und eines Elektrodenabstandes von di = d = 4mm ergibt sich dann an einem der Durchflußmesser eine induzierte Spannung von nur etwa 50 Mikrovolt. Die Spannungsdifferenz zwischen beiden Durchflußmessern ist noch mindestens um den Faktor 10 geringer, d.h. 5 Mikrovolt. Eine so geringe Meßspannung unter den bei einem Dialysegerät vorliegenden Bedingungen, d.h. an einem offenen, wegen der notwendigen Auswechselbarkeit aller mit dem Blut in Berührung kommenden Teile nur begrenzt abschirmbaren System mit ausreichender Genauigkeit zu verarbeiten, stellt ein schwieriges technisches Problem dar, das wahrscheinlich nur mit einem unvertretbar hohen Aufwand und mit erheblichen Einschränkungen für die Handhabung gelöst werden kann.

Ein induktiver Durchflußmesser hat unabhängig davon bei der Messung an strömendem Blut den Nachteil, daß das Blut über Elektroden mit elektrischen Stromkreisen in Verbindung gebracht werden muß, und daß zur Schaffung dieser Verbindungen zwischen dem nur zum einmaligen Gebrauch bestimmten, auswechselbaren Blutschlauchsystem und den Meßstromkreisen entsprechende elektrische Anschlußvorrichtungen vorgesehen werden müssen, deren Funktion durch den Betrieb in einer durch die Anwendung von Desinfektionsmitteln, Infusionslösungen u. dgl. stets feuchten Umgebung sehr gefährdet wäre.

Zur Messung des Blutflusses ist ferner das Ultraschall-Doppler-Verfahren bekannt. Dieses Verfahren hat prinzipiell den Vorteil, daß es sich um eine wirklich nicht-invasive Messung handelt, und daß insbesondere keine elektrischen Verbindungen zum Meßmedium hergestellt werden müssen. In dem Buch "Replacement of Renal Function by Dialysis" (Hrsg. W. Drukker et al., Verlag Martinus Nijhoff, den Haag, 1978) wird auf S. 267 dieses Verfahren zur Überwachung des Blutflusses bei der Hämodialyse in Betracht gezogen, jedoch insgesamt als zwar theoretisch vorteilhaft aber nach dem Stand der technischen Entwicklung wenig brauchbar bewertet. Eine auführlichere Bewertung eines handelssüblichen Gerätes zur Messung des Blutflusses nach dem Ultraschall-Doppler-Verfahren findet sich in dem Buch "Technical Aspects of Renal Dialysis" (Hrsg. T.H. Frost, Verlag Pittman Medical, Tunbridge Wells, 1978). Darin wird die Genauigkeit des Meßgerätes unter verschiedenen Einflüssen untersucht und als etwa vergleichbar mit der Luftblasen-Laufzeit-Methode eingestuft. Diese ist jedoch bekanntermaßen relativ ungenau, so daß auch aufgrund dieser Bewertung eigentlich nicht erwartet werden könnte, daß mit Hilfe von zwei nach diesem Prizip arbeitenden Durchfußmessern eine genügend genaue Ermittlung des Flüssigkeitsentzuges bei Hämodialyse- und Hämofiltrationsvorrichtungen möglich wäre.

Ein derartiges Gerät ist beispielsweise aus der DD-A- 244 816 bekannt, in der ein Durchflußsensor zur dopplersonografischen Messung mit einem in ein Blutschlauchsystem eingefügten formstabilen Meßeinsatz mit definiertem Strömungsquerschnitt und zeitlichen Ansätzen zur definierten Ein - und Auskopplung und Führung der Ultraschallwellen beschrieben ist. Diese aus mehreren Komponenten bestehende Sensoreinrichtung arbeitet jedoch dann ungenau, wenn die Messungen nicht unter stabilen Bedingungen durchgeführt werden. Dies ist beispielsweise dann der Fall, wenn Messungen an einem sich bewegenden Objekt (beispielsweise bei einem Patienten während einer extrakorporalen Blutbehandlung) vorgenommen werden.

Der Erfindung lag die Aufgabe zugrunde, die Vorrichtung der eingangs erwähnten Art so vorzubilden, daß reproduzierbare Messungen des Blutflusses mit erhöhter Genauigkeit durchgeführt werden können.

Der Erfindung lag weiterhin die Aufgabe zugrunde, eine Hämodialyse- und Hämofiltrationsvorrichtung mit einer funktionsfähigen und unter realen Betriebsbedingungen brauchbaren Einrichtung zur Messung des Flüssigkeitsentzuges durch Differenzbildung der Ausgangssignale zweier Durchflußmesser zu schaffen, von denen einer in die vom Patienten zur Dialysator- oder Filteranordnung führenden Blutleitung und der andere in die von der Dialysator- oder Filteranordnung zum Patienten führenden Blutleitung eingefügt ist.

Diese Aufgabe wird dadurch gelöst, daß der Durchflußsensor einen in das Blutschlauchsystem eingefügten formstabilen Meßeinsatz mit definiertem

Strömungsquerschnitt und mit dem Meßeinsatz verbundenen seitlichen Ansätzen zur definierten Ein-und Auskopplung und Führung der Ultraschallwellen aufweist.

Die erfindungsgemäße Anordnung weist den Vorteil auf, daß sie gegenüber den üblichen, nach dem Ultraschall-Doppler-Verfahren arbeitenden Meßanordnungen eine erheblich verbesserte Meßgenauigkeit aufweist. So können Meßfehlergrenzen für übliche Durchflußsensoren, die bei etwa 5% liegen, durch die erfindungsgemäße Vorrichtung erheblich gesenkt werden. So kann bspw. durch die erfindungsgemäße Vorrichtung eine Meßfehlergrenze von etwa 1% und darunter erreicht werden.

Gemäß einer bevorzugten Ausführungsform werden zwei Durchflußmesser der erfindungsgemäßen Art stromauf bzw. stromab einer Filtereinheit bzw. eines Dialysators im extrakorporalen Blutkreislauf angeordnet. Diese Durchflußmesser sind jeweils mit einer Auswerteeinheit verbunden, die gemäß einer vorbestimmten Rechenoperation einen Differenzwert zwischen den jeweiligen Signalen bildet. Da sich bei einer derartigen Differenzbildung die üblichen Meßfehler herausmitteln, kann der tatsächliche Meßfehler mit der erfindungsgemäßen Anordnung auf 0,1% und darunter gesenkt werden. Insofern läßt sich also die erfindungsgemäße Anordnung auch zur Feststellung geringster Änderungen der Durchflußmenge einsetzen, bspw. zur Einstellung geringer Ultrafiltrationsraten bei der Hämodialyse.

Unter Behandlung von Blut fällt erfindungsgemäß nicht nur eine Blutreinigung in Form der Hämodialyse oder -filtration, sondern auch jede Art von extrakorporaler Blutbehandlung, bei der der Blutfluß und der Blutdrucksatz überwacht werden und registriert werden sollen. So kann die erfindungsgemäße Anordnung auch im Herz-Lungen-Maschinen oder Plasmapheresezentrifugen verwendet werden.

Weitere Eigenschaften und Vorteile der Erfindung sowie weitere Ausgestaltungen ergeben sich aus der folgenden Beschreibung.

Von den zugehörigen Abbildungen zeigt:

Fig. 1      - ein Schema der Flüssigkeitskreisläufe einer Hämodialyse- und Hämofiltrationsvorrichtung entsprechend der Erfindung

Fig. 2      - ein zweites Ausführungsbeispiel in schematischer Darstellung

Fig. 3      - einen Sensor für die Durchflußmessung

Fig. 4      - eine Draufsicht des Meßeinsatzes für den Sensor

Fig. 5      - eine Seitenansicht des Meßeinsatzes für den Sensor

Fig. 6      - einen Sensor für die Durchflußmessung nach dem Ultraschall-Doppler-Verfahren bekannter Bauart in schematischer Darstellung zum Vergleich

Fig. 7      - ein Blockschema einer Schaltungsanordnung für die Signalverarbeitung

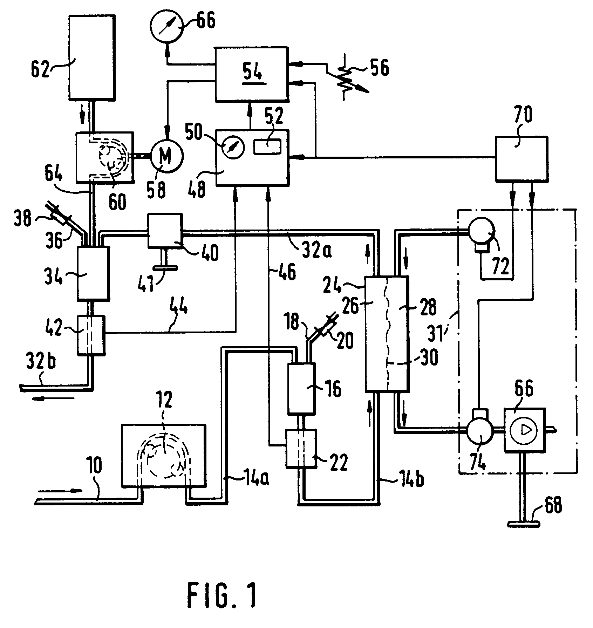

In Fig. 1 ist des Schema der Flüssigkeitskreisläufe einer Hämodialyse- und Hämofiltrationsvorrichtung entsprechend einem bevorzugten Ausführungsbeispiel der Erfindung dargestellt. Das dem Patienten z.B. über einen Katheter entnommene Blut gelangt durch die Leitung 10 zum Einlaß der Blutpumpe 12 und wird von dieser durch die Leitung 14a,14b der Dialysator- oder Filteranordnung 24 zugeführt. Diese umfaßt einen vom Blut durchströmten Raum 26 und einem durch eine semipermeable Membran 30 davon getrennten Raum 28 zur Durchströmung mit der Dialysierflüssigkeit und/oder Abführung von Filtrat, wobei das Filtrat (auch Ultrafiltrat genannt) unter dem Einfluß einer Druckdifferenz zwischen den Räumen 26 und 28, des sogenannten Transmembrandruckes, durch die semipermeable Membran 30 aus dem Raum 26 in den Raum 28 übergeht.

Die Einrichtungen zum Betrieb der Dialysator- oder Filteranordnung 24, z.B. zur Durchströmung mit Dialysierflüssigkeit und/oder Abführung von Filtrat, sind in der Betriebseinheit 31 zusammengefaßt. Diese ist in verschiedenen Ausführungen bekannt, die sämtlich für den vorliegenden Zweck geeignet sind. Sie kann z.B. aus einem Behälter mit Dialysierflüssigkeit und einer Umwälzpumpe bestehen, oder sie kann eine Einrichtung bekannter Art zur kontinuierlichen Erzeugung von Dialysierflüssigkeit aufweisen. Bei reinem Filtrationsbetrieb besteht sie normalerweise aus einer Saugvorrichtung und einem Sammelgefäß für das abgesaugte Filtrat. Im einfachsten Falle ist der Raum 28 nur mit einem Sammelgefäß verbunden, wobei das Filtrat lediglich unter dem Einfluß des im Raum 26 herrschenden Blutdruckes durch die Membran 30 in den Raum 28 übertritt und in dem Sammelgefäß aufgefangen wird.

Von der Dialysator- oder Filteranordnung 24 gelangt das Blut durch die Leitung 32a zu der Kammer 34, die in bekannter Weise u.a. der Abscheidung eventuell mitgeführter Luftblasen dient. Vom unteren Teil der Kammer 34 fließt das Blut durch die Leitung 32b zurück zum Patienten. In die Leitung 32b ist stromabwärts von der Kammer 34 der abflußseitige Durchflußsensor 42 eingefügt, dessen Gestaltung entsprechend der Erfindung weiter unten beschrieben wird. Er steht über die elektrische Leitung 44 mit der Auswertungseinheit 48 in Verbindung.

Entsprechend einer bevorzugten Ausgestaltung der Erfindung ist auch in die zur Dialysator- oder Filteranordnung führende Leitung 14a, 14b eine Kammer 16 und stromabwärts von dieser der zuflußseitige Durchflußsensor 22 eingefügt. Das Einfügen der Kammer 16 hat u.a. den Vorteil, daß Luft, die bei Auftreten einer Undichtigkeit im Bereich der Leitung 20 und des zugehörigen Patientenanschlusses in den Blutkreislauf eingesaugt werden kann, abgeschieden wird und somit nicht zu der Dialysator- oder Filteranordnung gelangt, wo sie sich, insbesondere bei den heute zumeist verwendeten Kapillardialysatoren oder -filtern, festsetzen und einen Teil der Kapillaren verstopfen oder zumindest den Strömungsverlauf stören könnte. Dies hätte eine Verminderung der Effektivität zur Folge und könnte darüber hinaus eine partielle Gerinnung des Blutes in der Dialysator- oder Filteranordnung fördern, so daß eine vollständige Rückspülung des Blutes am Ende der Behandlung nicht möglich wäre. Von besonderer Bedeutung ist das Einfügen der Kammer 16 in die Leitung 14a,14b und die Anordnung des Durchflußsensors 22 stromabwärts von der Kammer 16 jedoch darüber hinaus für die Funktion der Durchflußmessung. Wenn Luft durch Sensor geht, ist das von ihm erhaltene Meßsignal unbrauchbar, so daß die Messung gestört würde.

Die Kammern 34 und 16 sind mit Einrichtungen zum Entfernen angesammelter Luft ausgestattet. Gemäß Fig. 1 kann hierzu ein an den oberen Teil der Kammer angeschlossener Schlauch 36 bzw. 18 vorgesehen werden, der normalerweise mit einem Hahn oder einer Klemme 38 bzw. 20 verschlossen ist. Außerdem ist es zweckmäßig, die Kammern mit Einrichtungen zur Überwachung der angesammelten Luftmenge auszustatten, z.B. mit Füllstandssensoren. Diese sind in Fig. 1 nicht dargestellt. Ebenfalls nicht dargestellt ist eine Einrichtung zur Überwachung des Druckes in der rückführenden Blutleitung, die normalerweise an die Kammer 34 angeschlossen wird.

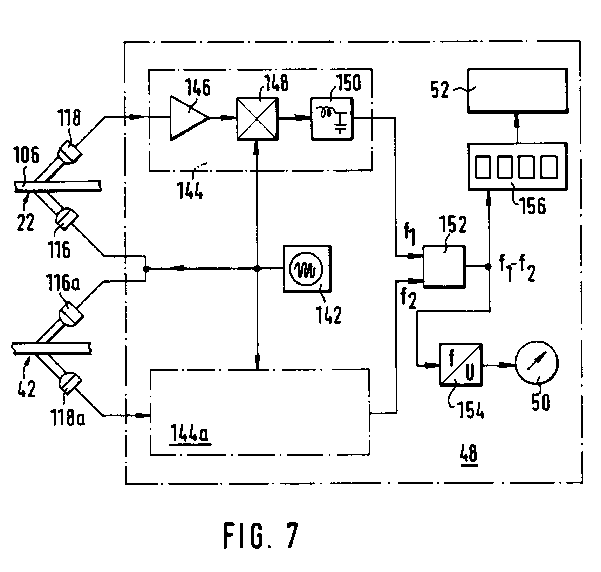

Die beiden Durchflußsensoren 22 und 42 stehen über elektrische Leitungen 46 bzw. 44 mit der Auswertungseinheit 48 in Verbindung. In dieser werden die von den Sensoren gelieferten Signale verarbeitet, u.a. zur Steuerung einer Anzeigevorrichtung 50 für die Netto-Flüssigkeitsentzugsrate, d.h. für die Flüssigmkeitsmenge, die dem Patienten pro Zeiteinheit entzogen wird, sowie einer Anzeigevorrichtung 52 für die entzogene Flüssigkeits-Gesamtmenge. Einzelheiten der Funktion der Auswertungseinheit werden weiter unten in Verbindung mit Fig. 7 geschrieben.

Eine weitere wichtige Ergänzung des bevorzugten Ausführungsbeispiels ist die Regelungseinheit 54. Sie steuert aufgrund eines Vergleichs zwischen dem an einer Einstellvorrichtung 56 vorgewählten Sollwert der Netto-Flüssigkeitsentzugsrate und dem durch die Auswertungseinheit 48 ermittelten Istwert der Netto-Flüssigkeitsentzugsrate den Antriebsmotor 58 der Infusionspumpe 60, und zwar in der Weise, daß die Antriebsgeschwindigkeit zunimmt, wenn der Istwert der Netto-Flüssigkeitsentzugsrate den Sollwert überschreitet. Die Infusionspumpe fördert aus einer Versorgungseinrichtung, z.B. einem Vorratsbehälter 62, eine Infusionslösung durch die Leitung 64 in die Kammer 34, wo sie sich mit dem zum Patienten zurückgeleiteten Blut vermischt. Die pro Zeiteinheit zugeführte Flüssigkeitsmenge wird außerdem auf der Anzeigevorrichtung 66 angezeigt.

Zum Betrieb der in Fig. 1 gezeigten Anordnung wird an der Einstellvorrichtung 56 zunächst der gewünschte Sollwert der Netto-Flüssigkeitsentzugsrate eingestellt. Solange aufgrund des Transmembrandruckes in der Dialysator- oder Filteranordnung die aus dem Blutraum 26 durch die Membran 30 in den Raum 28 übergehende Flüssigkeitsmenge pro Zeiteinheit geringer ist als der Sollwert der Netto-Flüssigkeitsentzugsrate, steht die Infusionspumpe entsprechend den Eigenschaften der Regelungseinheit 54 still, da in diesem Zustand der Istwert der Netto-Flüssigkeitsentzugsrate kleiner ist als der Sollwert. Nun wird der Transmembrandruck durch Erhöhung des Druckes im Raum 26 und/oder Absenkung des Druckes im Raum 28 erhöht. Die Druckerhöhung im Raum 26 kann mittels einer an der Leitung 32a angeordneten Drossel 40 bewirkt werden, die Druckabsenkung im Raum 28 ebenfalls durch Einstellung eines entsprechenden Drosselventils oder durch Erhöhung der Saugleistung einer zu der Betriebseinheit 31 gehörigen Saugvorrichtung 66, z.B. mit einer Pumpe, bewirkt werden.

Sobald durch den gesteigerten Transmembrandruck die pro Zeiteinheit durch die Membran 30 tretende Flüssigkeitsmenge den eingestellten Sollwert der Netto-Flüssigkeitsentzugsrate übersteigt, tritt die Infusionspumpe 60 in Funktion, und aufgrund der Eigenschaften der Regelungseinheit 54 wird durch die Infusionspumpe 60 pro Zeiteinheit so viel Infusionsflüssigkeit zugeführt, wie notwendig ist, um den den Sollwert der Netto-Flüssigkeitsentzugsrate übersteigenden Teil der durch die Membran 30 pro Zeiteinheit ausgeschiedenen Flüssigkeitsmenge zu ersetzen. Ausgehend von einer bestimmten Geschwindigkeit des Blutzuflusses, die durch den Sensor 22 erfaßt wird, wird also im Gleichgewichtszustand durch die Regelung des Antriebes der Infusionspumpe 60 von dieser so viel Flüssigkeit zugeführt, daß sich die von dem zweiten Sensor 42 erfaßte Durchflußgeschwindigkeit des zum Patienten zurückfließenden Blutes um den vorgesehenen Differenzbetrag, entsprechend der an der Einstellvorrichtung 56 vorgewählten Netto-Flüssigkeitsentzugsrate, unterscheidet.

An der Anzeigevorrichtung 66 ist die pro Zeiteinheit zugeführte Menge der Infusionsflüssigkeit ablesbar. Unter Beobachtung dieser Anzeige kann der Transmembrandruck mit Hilfe der Einstellvorrichtungen 41 und/oder 68 so einreguliert werden, daß die pro Zeiteinheit zugeführte Menge Infusionsflüssigkeit in der gewünschten Größenordnung liegt.

Störungen im Blutkreislauf oder in der Betriebseinheit 31 führen im allgemeinen zu einer Veränderung der Transmembrandruckes und beeinflussen somit den Durchtritt von Flüssigkeit durch die Membran 30. Aufgrund der beschriebenen Eigenschaften des Regelkreises führt dies automatisch zu einer Korrektur in Form einer entsprechenden Veränderung der Flüssigkeitszufuhr durch die Infusionspumpe 60 und des auf dem Anzeigeinstrument 66 angezeigten Wertes. Durch Verwendung eines Anzeigeinstruments 66 mit einstellbaren Alarm-Grenzkontakten, wie in Fig. 1 angedeutet, oder gleichwertige Einrichtung zur Grenzwertüberwachung besteht somit die Möglichkeit, eine derartige Störung selbsttätig zu erkennen. Dies hat den wesentlichen Vorteil, daß die sonst zur Erkennung solcher Störungen üblichen, sehr aufwendigen und störanfälligen Druckmeßeinrichtungen zur Überwachung des Transmembrandruckes bei diesem System entfallen können.

In weiterer Ausgestaltung sieht die Erfindung eine Einrichtung zur selbsttätigen periodischen Kalibrierung der Durchflußsensoren in Verbindung mit der Auswertungseinheit 48 vor. Sie umfaßt die Kalibrier-Steuerungseinheit 70 und die von dieser gesteuerten Ventile 72 und 74, die zum Zwecke der Kalibrierung durch das entsprechende Steuerungssignal geschlossen werden. Gleichzeitig wird der Antrieb der Infusionspumpe 60 angehalten. Das Schließen der genannten Ventile dient dazu, den Raum 28 der Dialysator-oder Filteranordnung 24 abzusperren, so daß ein weiterer Durchtritt von Filtrat aus dem Raum 26 durch die Membran 30 in den Raum 28 verhindert ist. Unter diesen Bedingungen muß in den beiden Durchflußsensoren 22 und 42 der gleiche Durchfluß herrschen. Sofern eine Abweichung zwischen den beiden von den Durchflußsensoren gelieferten Meßwerten besteht, wird diese in der Auswertungseinheit 48 ermittelt und gespeichert und in der auf die Kalibrierung folgenden Betriebszeit zur Fehlerkorrektur verwendet. Auf diese Weise wird eine Nullpunktkorrektur bezüglich der Differenzbildung zwischen den Meßsignalen der Durchflußsensoren erreicht und somit der hauptsächliche mögliche Fehlereinfluß beseitigt.

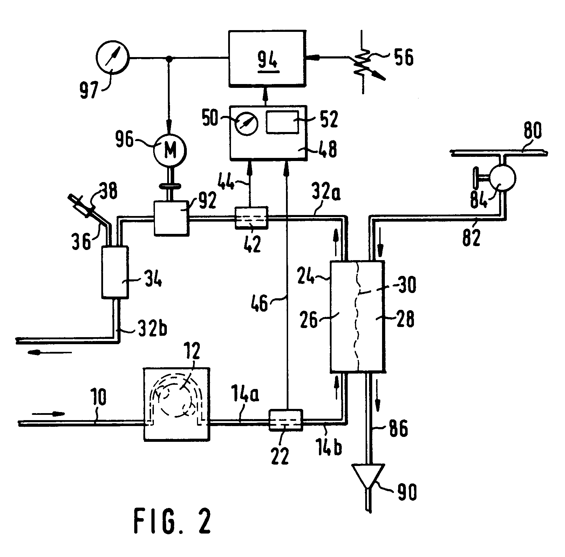

Ein anderes Ausführungbeispiel einer Hämodialyse- und Hämofiltrationsvorrichtung entsprechend der Erfindung ist in Fig. 2 schematisch dargestellt. Zur Durchströmung des Raumes 28 der Dialysator- oder Filteranordnung 24 ist dieser einerseits an eine Verteilerleitung 80 eines zentralen Versorgungssystems für Dialysierflüssigkeit angeschlossen, andererseits an einen Abluß 90. Der Durchfluß wird mit einen Hahn 84 eingestellt. Bei reinem Filtrationsbetrieb bleibt der Hahn 84 geschlossen.

Der extrakorporale Blutkreislauf umfaßt zwei Durchflußsensoren 22 und 42 in der zuführenden Leitung 14a,14b bzw. in der abführenden Leitung 32a,32b des Raumes 26 der Dialysator- oder Filteranordnung 24. Sie sind wie bei dem vorhergehenden Ausführungsbeispiel über elektrische Leitungen 44 und 46 mit der Auswertungseinheit 48 verbunden, die eine Anzeigevorrichtung 50 für die Differenz der von den beiden Durchflußsensoren gelieferten Signale aufweist. Diese Differenz entspricht wegen der für dieses Ausführungsbeispiel bevorzugten Anordnung der Durchflußsensoren unmittelbar stromaufwärts und stromabwärts von dem Raum 26 der Dialysator- oder Filteranordnung derjenigen Flüssigkeitsmenge pro Zeiteinheit, die durch Filtration aus dem Raum 26 durch die Membran 30 in den Raum 28 übergeht. Diese entspricht zugleich der Flüssigkeitsmenge, die dem Patienten pro Zeiteinheit entzogen wird. Zur Anzeige der bis zum jeweiligen Zeitpunkt insgesamt entzogenen Flüssigkeitsmenge enthält die Auswertungeinheit 48 eine weitere Anzeigevorrichtung 52.

Die pro Zeiteinheit aus dem Raum 26 durch die Membran 30 in den Raum 28 übergehende Flüssigkeitsmenge ist von der Druckdifferenz zwischen den Räumen 26 und 28 abhängig. Zur Einstellung dieser Druckdifferenz ist an der abführenden Blutleitung eine einstellbare Drossel 92 vorgesehen. Im einfachsten Falle wird die Drossel 92 unter Beobachtung der Anzeigevorrichtung 50 von Hand so eingestellt, daß der gewünschte Flüssigkeitsentzug pro Zeiteinheit erreicht wird.

Es ist jedoch auch die Möglichkeit vorgesehen, die Drossel 92 durch eine Regelung selbsttätig einzustellen. Hierzu dient die Regelungseinheit 94, die mit der Einstellvorrichtung 56 zur Vorwahl des Sollwertes des Flüssigkeitsentzuges pro Zeiteinheit verbunden ist. Die Reglungseinheit vergleicht diesen mit dem von der Auswertungseinheit 48 ermittelten Istwert und steuert entsprechend dem Ergebnis dieses Vergleiches einen Motor 96 zum Verstellen der Drossel 92.

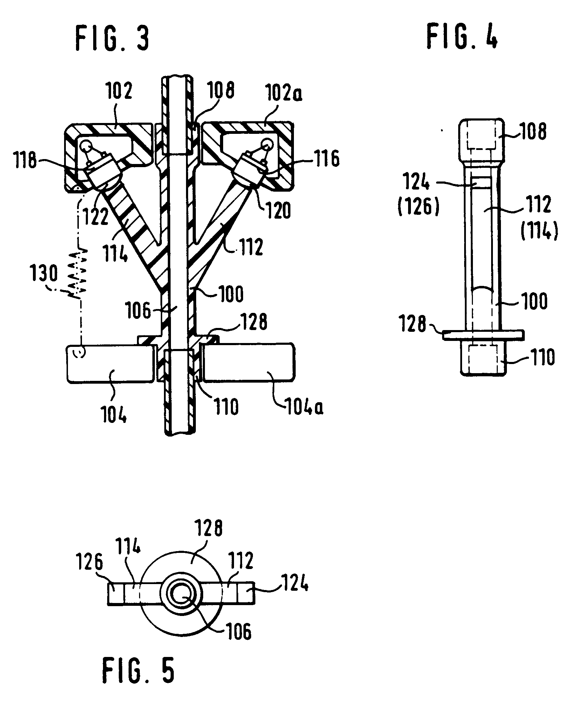

Von wesentlicher Bedeutung für die Erfindung ist die Bauart der für die Durchflußmessung eingesetzten Sensoren 22,42. Sie ist in Fig. 3 dargestellt. Die Abbildung ist nur ein Schema, das die hauptsächlichen Bestandteile und ihr Zusammenwirken veranschaulichen soll. Die gesamte Anordnung besteht im wesentlichen aus einem Meßeinsatz 100, der zu dem auswechselbaren und im allgemeinen nur zum einmaligen Gebrauch bestimmten Blutschlauchsystem gehört, und einer zum festen Bestandteil des Gerätes gehörenden Halterung 102,104, in der der Meßeinsatz befestigt wird. Die Halterung besteht vorzugsweise aus zwei Teilen, die zur Fixierung des Meßeinsatzes federnd miteinander verbunden sind. Eine Seitenansicht und eine Draufsicht des Meßeinsatzes sind in Fig. 4 bzw. Fig. 5 dargestellt.

Der Meßeinsatz besteht aus einem blutverträglichen, formstabilen und relativ starren Kunststoffmaterial, das sich nach dem üblichen Spritzgießverfahren zu Teilen hoher Maßgenauigkeit und Gleichmäßigkeit verarbeiten läßt. Der Meßeinsatz wird vorzugsweise schon im Herstellerwerk in das Blutschlauchsystem eingefügt, z.B. durch das auch für ähnliche Teile übliche Einkleben der betreffenden Schlauchabschnitte in die Anschlußteile 108,110 des Meßeinsatzes. Der Meßeinsatz wird mit dem Schlauchsystem sterilisiert, und das gesamte zum einmaligen Gebrauch bestimmte System gelangt so zur Verwendungsstelle, wo es mit der übrigen Apparatur verbunden wird.

Der Meßeinsatz hat in Fortsetzung der angeschlossenen Leitungteile einen durchgehenden Strömungskanal 106. Von besonderer Wichtigkeit sind die beiden seitlichen Ansätze 112,114, die auf den Strömungskanal gerichtet sind und der Ein- bzw. Auskopplung des Ultraschallsignals dienen und außerdem die Funktion haben, die Ultraschallwellen zu führen, d.h. die Ausbreitungsrichtung der eingekoppelten Ultraschallwellen genau zu definieren und ebenso die Richtung der maximalen Intensität der auskoppelten Ultraschallwellen exakt festzulegen. Die seitlichen Ansätze stehen zur Längsachse des Strömungskanals in einem Winkel z.B. in der Größenordnung von 15-50 Grad. Im Interesse einer hohen Empfindlichkeit ist ein relativ kleiner Winkel vorteilhaft.

Im oberen Teil 102,102a der Halterung sind die beiden Ultraschallwandler für die Aussendung und den Empfang des Ultraschallsignals angeordnet, und zwar so, daß ihre Achsen die gleiche Richtung wie die der Führung der Ultraschallwellen dienenden Ansätze des Meßeinsatzes haben. Sie bestehen im wesentlichen aus je einem Wandlerelement 116,118 und einem Ankopplungsstück 120, 122. Die Ankopplungsstücke stehen unter einem gewissen Druck mit den Kopplungsflächen 124,126 des Meßeinsatzes in inniger Berührung. Die Wandlerelemente sind vorzugsweise Scheiben aus einem piezoelektrischen keramischem Material, die in üblicher Art mit Metallbelägen und elektrischen Anschlüssen ausgestattet sind. Die Ankopplungsstücke bestehen zumindest in dem Teil, der mit den Kopplungsflächen in Berührung kommt, vorzugsweise aus einem elastischen Material, z.B. Silikonkautschuk, so daß sich unter der Wirkung des Anpreßdruckes eine weitgehend homogene und ausreichend große Berührungsfläche ausbildet, die sich möglichst über die gesamte Kopplungsfläche 124,126 erstreckt. Dadurch wird es überflüssig, zur Verbesserung der Ankopplung besondere Kontaktgele, wie sonst vielfach üblich, zu benutzen.

Der untere Teil 104,104a der Halterung hat im wesentlichen die Funktion, den Meßeinsatz 100 zu fixieren. Er ist zu diesem Zweck in einer geeigneten Führung federnd gegenüber dem oberen Teil 102,102a der Halterung gelagert, wie dies in Fig. 3 durch eine Zugfeder 130 rein schematisch angedeutet ist. Selbstverständlich muß hierbei die resultierende Kraft im der Symmetrieachse der Anordnung wirken, was z.B. durch die symmetrische Anordnung von zwei Federn zu erreichen wäre. Der Meßeinsatz weist zu dem gleichen Zweck ein z.B. kragenförmig ausgebildetes Stützelement 128 auf, durch das die Kraft von dem unteren Teil der Halterung auf den Meßeinsatz übertragen wird.

Durch die oben beschriebene definierte Ein- und Auskopplung und Führung der Ultraschallwellen werden die Voraussetzungen für eine hohe Genauigkeit bei der Messung der Strömungsgeschwindigkeit geschaffen. Von erheblicher Bedeutung ist außerdem, daß durch die Gestaltung des Meßeinsatzes das Blut vor und hinter der eigentlichen Meßstelle über eine längere Strecke, die ein Mehrfaches des Durchmessers des Strömungskanals beträgt, in einem definierten, gerade verlaufenden Strömungskanal geführt wird, so daß Störungen der Strömungsprofils durch Leitungskrümmungen vermieden werden.

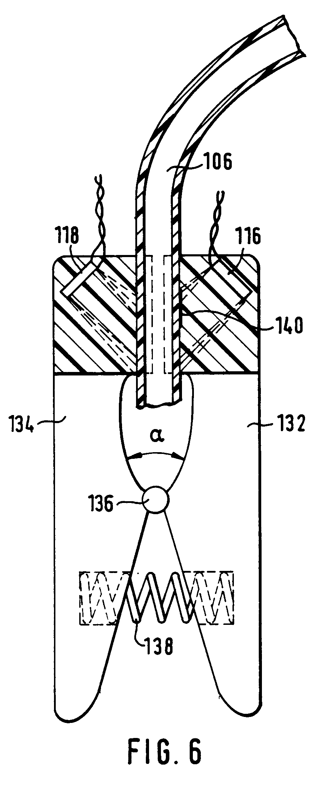

Zum Vergleich und zur Verdeutlichung der Unterschiede ist in Fig. 6 die Konstruktion des Durchflußsensors eines bekannten Blutflußmeßgerätes schematisch dargestellt, auf das sich auch die bereits erwähnte Veröffentlichung in dem dem Buch "Technical Aspects of Renal Dialysis" (Hrsg. T.H. Frost, Verlag Pittman Medical, Tunbridge Wells, 1978) bezieht. Der Sensor ist hierbei nach Art einer Klammer ausgebildet, die auf den Blutschlauch geklemmt wird. Hierzu sind die beiden Wangen der Klammer durch ein Gelenk 136 und eine Feder 138 verbunden. Der vordere Teil der Wangen ist in seiner inneren Kontur den Maßen des Blutschlauches angepaßt und umschließt diesen. Die Wandlerelemente 116,118 sind im vorderen Teil der Wangen angeordnet und unter einem Winkel von ca. 45 Grad auf den Blutschlauch ausgerichtet und durch Einbettung in eine Vergußmasse fixiert.

Eine solche Anordnung hat verschiedene Nachteile, die bei der zuvor beschriebenen vermieden sind:

- Der Winkel (alpha) zwischen den Wangen ändert sich in Abhängigkeit von den Toleranzen des Schlauch-Außendurchmessers sowie bei Druckänderungen im Schlauch und bei temperaturabhängiger Veränderung der Elastizität des Schlauchmaterials. Durch die Veränderung dieses Winkels verändert sich auch der Anstellwinkel der Wandler in Richtung auf den Strömungskanal und damit der für die Kalibrierung maßgebende Proportionalitätsfaktor.

- Veränderungen der lichten Weite des Schlauches zwischen den Wangen, z.B. infolge von Druckschwankungen oder temperaturabhängiger Veränderung der Elastizität des Schlauchmaterials in Verbindung mit der einwirkenden Federkraft, beeinflussen ebenfalls die Länge der von den Ultraschallwellen im Blut zurückgelegten Strecke und führen somit ebenfalls zu Veränderungen des Meßsignals.

- Die Haupt-Ausbreitungsrichtung der Ultraschallwellen vom sendeseitigen Wandler in den Strömungskanal einerseits und die Richtung der maximalen Empfindlichkeit für die vom Strömungskanal zum emfängerseitigen Wandler gelangenden Ultraschallwellen andererseits wird von den Übertragungsverhältnissen an der Grenzfläche zwischen der inneren Kontur der Wange und der Außenfläche des Schlauches beeinflußt. Eine ungleichmäßige Druckverteilung und/oder ein unvollständiger Flächenkontakt, insbesondere aber Schmutzpatikel oder Luftblasen (140 in Fig. 6), die in das zur Verbesserung der Ankopplung verwendete Gel eingeschlossen sein können, verändern somit ebenfalls in unvorhersehbarer Weise den für die Kalibrierung maßgebenden Proportionalitätsfaktor.

- Die innere Querschnittsfläche von Schläuchen unterliegt, da diese üblicherweise nach einem Extrusionsverfahren hergestellt werden, erheblichen Toleranzen. Aufgrund des Zusammenhanges zwischen Durchfluß, mittlerer Strömungsgeschwindigkeit und Querschnittsfläche führt dies zu einer entsprechenden zusätzlichen Meßunsicherheit.

- Da der Strömungskanal beiderseits der Meßstelle keine vorbestimmte Richtung hat, ergeben sich durch Verlagerung, z.B. Krümmung, des Schlauches Veränderungen des Strömungsprofils die zu entsprechenden Fehlern führen.

Im Vergleich zu einem induktiven Durchflußmesser, bei dem nicht nur der Strömungsquerschnitt, sondern auch die magnetische Flußdichte und der Elektrodenabstand mit hoher Genauigkeit eingehalten werden müssen, beschränken sich diese hohen Anforderungen bei dem Gegenstand der Erfindung unter der Voraussetzung einer exakten Führung der Ultraschallwellen im wesentlichen auf die Genauigkeit des Strömungsquerschnitts und der Anregungsfrequenz. Durch die Herstellung des Meßeinsatzes als Präzisions-Spritzteil bzw. Anwendung eines quarzgesteuerten Generators für die Schwingungsanregung können beide Anforderungen mit geringem Aufwand erfüllt werden. Ein weiterer Vorteil der Durchflußmessung nach dem Ultraschall-Doppler-Prinzip gegenüber der induktiven Durchflußmessung ergibt sich daraus, daß die Amplitude der Meßsignale eine untergeordnete Rolle spielt. Der Meßeffekt liegt vielmehr in einer Frequenzänderung, die relativ leicht fehlerfrei ausgewertet werden kann.

In Fig. 7 ist ein Blockschema einer Schaltungsanordnung für den Betrieb der Durchflußsensoren 22 und 42 angegeben, das der Auswertungsschaltung 48 in Fig. 1 und Fig. 2 entspricht. Die senderseitigen Ultraschallwandler 116,116a werden gemeinsam von einem Hochfrequenzgenerator 142 mit einer Frequenz in der Größenordnung von einigen MHz gespeist. Die vom empfängerseitigen Ultraschallwandlern 118,118a gelieferten Signale enthalten neben Anteilen der Sendefrequenz das eigentliche Meßsignal in Form eines Schwingungsanteils, der aufgrund des Dopplereffekts eine der Strömungsgeschwindigkeit proportionale Frequenzverschiebung aufweist. Die Verarbeitung dieser Signale erfolgt getrennt in zwei gleichen Schaltugseinheiten 144,144a. Das Signal wird zunächst durch den Verstärker 146 auf eine ausreichende Amplitude gebracht und einer Mischstufe 148 zugeführt, um die der Strömungsgeschwindigkeit proportionale niederfrequente Differenzfrequenz f1 bzw. f2 zu gewinnen. Diese steht am Ausgang des nachgeschalteten Tiefpasses 150, getrennt von den übrigen Freuenzanteilen, zur Verfügung. In einer weiteren Schaltungseinheit 152 wird aus f1 und f2 schließlich die Differenz f1-f2 gebildet, die für den Unterschied der Durchflüsse in den beiden Durchflußsensoren und somit für den Flüssigkeitsentzug pro Zeiteinheit maßgebend ist.

Zur Anzeige dieser Größe kann das Signal f1-f2 z.B. durch einen Frequenz-Spannungs-Wandler 154 in eine Spannung umgesetzt und damit das Anzeigeinstrument 50 gesteuert werden. Zur Anzeige der Gesamtmenge der entzogenen Flüssigkeit wird das Signal f1-f2 einem Zähler 156 zugeführt und das jeweilige Zählergebnis auf der Anzeigevorrichtung 52 angezeigt.

## Patentansprüche

1. Vorrichtung zur extrakorporalen Behandlung von Blut, insbesondere zur Hämodialyse oder Hämofiltration, mit einem extrakorporalen Blutkreislauf, der durch ein Blutschlauchsystem gebildet wird, in das mindestens ein nach dem Ultraschall-Prinzip arbeitender Durchflußsensor eingefügt ist, dadurch gekennzeichnet, daß der Durchflußsensor (22, 42) einen in das Blutschlauchsystem eingefügten formstabilen Meßeinsatz (100) mit definiertem Strömungsquerschnitt und mit dem Meßeinsatz (100) einteilig verbundenen seitlichen Ansätzen (112, 114) zur definierten Ein- und Auskopplung und Führung der Ultraschallwellen aufweist.

2. Vorrichtung nach Anspruch 1, dadurch gekennzeichnet, daß stromauf und stromab einer Blutbehandlungseinrichtung (24) jeweils Durchflußsensoren (22, 42) angeordnet sind, die auf dem Ultraschalldopplereffekt basieren und den Meßeinsatz (100) gemäß Anspruch 1 aufweisen, und daß die Durchflußsensoren (22, 42) mit einer Auswertungseinheit (48) verbunden sind, die aus den von den Durchflußsensoren (22, 42) übermittelten Signalen eine vorbestimmte Auswertungsoperation vornimmt.

3. Vorrichtung nach Anspruch 2, dadurch gekennzeichnet, daß die beiden Durchflußsensoren (22, 42) mit einer Auswertungseinheit (48) verbunden sind, die eine Einrichtung (152) zur Ermittlung der Differenz der Durchflüsse und eine Anzeigevorrichtung (50) zur Anzeige der dem Patienten pro Zeiteinheit entzogenen Flüssigkeitsmenge aufweist.

4. Vorrichtung nach einem der vorhergehenden Ansprüche 2 oder 3, gekennzeichnet durch eine mit den Durchflußsensoren (22, 42) verbundene Auswertungseinheit (48), die Einrichtungen (152, 156) zur Bildung des Zeitintegrals der Differenz der Durchflüsse und eine Anzeigevorrichtung (52) für die dem Patienten insgesamt entzogene Flüssigkeitsmenge aufweist.

5. Vorrichtung nach Anspruch 3 oder 4, gekennzeichnet durch eine mit der Auswertungseinheit (48) verbundene Regelungseinheit (54) zur selbsttätigen Regelung der Zufuhr einer Infusionslösung in den extrakorporalen Blutkreislauf.

6. Vorrichtung nach Anspruch 5, dadurch gekennzeichnet, daß die Regelungseinheit (54) mit einer Anzeigevorrichtung (56) zur Anzeige und Grenzwertüberwachung der Zufuhrgeschwindigkeit der Infusionslösung verbunden ist.

7. Vorrichtung nach einem der vorhergehenden Ansprüche 2 bis 6, gekennzeichnet durch eine mit der Auswertungseinheit (48) verbundene Regelungseinheit (94) zur selbsttätigen Einstellung des Transmembrandruckes.

8. Vorrichtung nach Anspruch 7, dadurch gekennzeichnet, daß die Regelungseinheit (94) zur selbsttätigen Einstellung des Transmembrandruckes mit einem Antriebsmotor (96) zur Betätigung einer an der rückführenden Blutleitung (32a, 32b) angeordneten Drossel (92) verbunden ist.

9. Vorrichtung nach Anspruch 7 oder 8, dadurch gekennzeichnet, daß die Regelungseinheit (94) mit einer Anzeigevorrichtung (97) zur Anzeige und Grenzwertüberwachung der der Einstellung des Transmembranabdruckes dienenden Größe verbunden ist.

10. Vorrichtung nach Anspruch 7 und 8, dadurch gekennzeichnet, daß die Anzeigevorrichtung (97) zur Anzeige und Grenzwertüberwachung der Stellung des Antriebsmotors (96) ausgebildet ist.

11. Vorrichtung nach einem der vorhergehenden Ansprüche 2 bis 10, dadurch gekennzeichnet, daß sie eine Einrichtung zur selbsttätigen periodischen Kalibrierung aufweist, wobei durch eine Kalibrier-Steuerungseinheit (70) der zur Durchspülung mit Dialysierflüssigkeit und/oder Abführung von Filtrat bestimmte Raum (28) der Dialysator- oder Filteranordnung (24) mittels Ventilen (72, 74) abgesperrt und die sich dabei einstellende Differenz der Meßsignale der beiden Durchflußsensoren (22 42) durch die Auswertungseinheit ermittelt wird, und daß die Auswertungseinheit Einrichtungen zur Speicherung dieser Differenz und zur Nullpunktkorrektur entsprechend der Größe dieser Differenz für das in der auf die Kalibrierung folgenden Betriebsphase gebildete Differenzsignal aufweist.

12. Vorrichtung nach einem der vorhergehenden Ansprüche 2 bis 11, dadurch gekennzeichnet, daß die Längsachsen der seitlichen Ansätze (112, 114) des Meßeinsatzes (100) mit der Längsachse des im Meßeinsatz verlaufenden Strömungskanals (106) einen Winkel in der Größenordnung von 10 bis 60°, vorzugsweise 30°, bilden.

13. Vorrichtung nach einem der vorhergehenden Ansprüche 2 bis 12, dadurch gekennzeichnet, daß die Halterung (102, 102a; 104, 104a) für den Meßeinsatz (100) Ultraschallwandler mit piezoelektrischen Wandlerelementen (116, 118) und mit diesen verbundene Ankopplungsstücke (120, 122) aufweist, die mit den an den Enden der seitlichen Ansätze (112, 144) befindlichen Kopplungsflächen (124, 126) unter Druck in Berührung stehen.

14. Vorrichtung nach Anspruch 13, dadurch gekennzeichnet, daß der mit den Kopplungsflächen (124, 126) in Berührung stehende Teil der Ankopplungsstücke aus einem gummielastischen Material besteht.

15. Vorrichtung nach Anspruch 13, dadurch gekennzeichnet, daß die Halterung für den Meßeinsatz (100) aus einem die Ultraschallwandler enthaltenden feststehenden Teil (102, 102a) und einem diesem gegenüber federnd gelagerten Teil (104, 104a) zur Fixierung des Meßeinsatzes und zur Erzeugung der an den Kopplungsflächen wirksamen Anpreßkraft besteht.

16. Vorrichtung nach Anspruch 13, dadurch gekennzeichnet, daß die zur Erzeugung des Ultraschalls dienenden senderseitigen Ultraschallwandler (116, 116a) von dem gleichen Hochfrequenzgenerator (142) gesteuert werden.

## Claims

1. Apparatus for the extracorporeal treatment of blood, in particular for hemodialysis or hemofiltration, comprising an extracorporeal blood circuit which is formed by a blood tubing system into which at least one flow sensor operating by the ultrasonic principle is inserted, characterized in that the flow sensor (22, 42) comprises a dimensionally stable measuring insert (100) inserted into the blood tubing system and having defined flow cross-section and lateral extensions (112, 114) which are integrally connected to the measuring insert for the defined coupling-in and coupling out and conducting of the ultrasonic waves.

2. Apparatus according to claim 1, characterized in that upstream and downstream of a blood treatment arrangement (24) respectively flow sensors (22, 42) are arranged which are based on the ultrasonic Doppler effect and having the measuring insert (100) according to claim 1, and that the flow sensors (22, 42) are connected to an evaluating unit (48) which effects a predetermined evaluating operation on the basis of the signals submitted by the flow sensors (22, 42).

3. Apparatus according to claim 2, characterized in that the two flow sensors (22, 42) are connected to an evaluating unit (48) which comprises a means (152) for determining the difference of the flows and a display means (50) for indicating the amount of fluid withdrawn from the patient per unit of time.

4. Apparatus according to one of the preceding claims 2 or 3, characterized by an evaluating unit (48) connected to the flow sensors (22, 42) and having means (152, 156) for forming the time integral of the difference of the flows and a display means (52) for the total fluid amount withdrawn from the patient.

5. Apparatus according to claim 3 or 4, characterized by a control unit (54) connected to the evaluating unit (48) for automatic regulation of the supply of an infusion solution into the extracorporeal blood cycle.

6. Appartus according to claim 5, characterized in that the control unit (54) is connected to a display means for indicating and limit-value monitoring of the supply rate of the infusion solution.

7. Apparatus according to any of the preceding claims 2 to 6, characterized by a control unit (94) connected to the evaluating unit (48) for automatic setting of the transmembrane pressure.

8. Apparatus according to claim 7, characterized in that the control unit (94) for automatic setting of the transmembrane pressure is connected to a drive motor (96) for actuating a throttle (92) arranged at the return blood conduit (32a, 32b).

9. Apparatus according to claim 7 or 8, characterized in that the control unit (94) is connected to a display means (97) for indicating and limit-value monitoring of the quantity serving for the setting of the transmembrane pressure.

10. Apparatus according to claim 9, characterized in that the display means (97) is constructed for displaying and limit-value monitoring of the position of the drive motor (96).

11. Apparatus according to any of the preceding claims 2 to 10, characterized in that it comprises a means for automatic periodic calibration wherein by a calibration control unit (70) the space (28) of the dialyzer or filter arrangement (24) intended for flushing with dialysis solution and/or withdrawal of filtrate is shut off by means of valves (72, 74) and the resulting differences of the measurement signals of the two flow sensors (22, 42) is determined by the evaluating unit, and that the evaluating unit comprises means for storing said difference and zero point correction corresponding to the magnitude of said difference for the difference signal formed in the operating phase following the calibration.

12. Apparatus according to claim 2, characterized in that the longitudinal axes of the lateral extensions (112, 114) of the measuring insert (100) form with the longitudinal axis of the flow passage (106) extending in the measuring insert an angle of the order of magnitude of 10 to 60°, preferably 30°.

13. Apparatus according to any of the preceding claims 2 to 12, characterized in that the holder (102, 102a; 104, 104a) for the measuring insert (100) comprises ultrasonic transducers with piezoelectric transducer elements (116, 118) and coupling pieces (120, 122) which are connected thereto and are in contact under pressure with the coupling faces (124, 126) disposed at the ends of the lateral extensions (112, 144).

14. Apparatus according to claim 13, characterized in that the part of the coupling pieces in contact with the coupling faces (124, 126) consists of rubber-elastic material.

15. Apparatus according to claim 13, characterized in that the holder for the measuring insert (100) consists of a fixed part (102, 102a) containing the ultrasonic transducers and a part (104, 104a) mounted resiliently with respect to said fixed part for fixing the measuring insert and for generating the pressure force acting at the coupling faces.

16. Apparatus according to claim 13, characterized in that the transmitter-side ultrasonic transducers (116, 116a) serving to generate the ultrasonic radiation are controlled by the same high-frequency generator (142).

**Revendications**

1. Dispositif pour le traitement extracorporel de sang, en particulier pour l'hémodialyse ou l'hémofiltration, comprenant un circuit sanguin extracorporel, formé par un système de tuyauterie sanguine, dans lequel est interposé au moins un capteur de débit fonctionnant selon le principe des ultrasons, caractérisé en ce que le capteur de débit (22, 42) comporte un insert de mesure (100) de forme stable et ayant une section d'écoulement définie, qui est interposé dans le système de tuyauterie sanguine et est pourvu d'appendices latéraux (112, 114), reliés d'un seul tenant à l'insert de mesure (100), pour assurer des couplages d'entrée et de sortie et un guidage définis des ondes ultrasoniques.

2. Dispositif selon la revendication 1, caractérisé en ce que des capteurs de débit (22, 42) basés sur l'effet Doppler à ultrasons et comportant un insert de mesure (100) selon la revendication 1, sont placés en amont et en aval d'un dispositif (24) de traitement du sang, et que les capteurs de débit (22, 42) sont connectés à une unité d'exploitation (48) qui effectue une opération d'exploitation prédéterminée sur les signaux transmis par les capteurs de débit (22, 42).

3. Dispositif selon la revendication 2, caractérisé en ce que les deux capteurs de débit (22, 42) sont connectés à une unité d'exploitation (48) comportant un dispositif (152) pour déterminer la différence des débits et un organe d'affichage (50) pour afficher la quantité de liquide retiré par unité de temps au patient.

4. Dispositif selon une des revendications 2, 3 précédentes, caractérisé par une unité d'exploitation (48) connectée aux capteurs de débit (22, 42) et comportant des dispositifs (152, 156) pour former l'intégrale par rapport au temps de la différence des débits, ainsi qu'un organe d'affichage (52) de la totalité du liquide retiré au patient.

5. Dispositif selon la revendication 3 ou 4, caractérisé par une unité de régulation (54) connectée à l'unité d'exploitation (48) et servant à la régulation automatique de l'apport d'un soluté de perfusion dans le circuit sanguin extracorporel.

6. Dispositif selon la revendication 5, caractérisé en ce que l'unité de régulation (54) est connectée à un organe d'affichage (56) pour afficher et surveiller les valeurs limites de la vitesse d'apport du soluté de perfusion.

7. Dispositif selon une des revendications 2 à 6 précédentes, caractérisé par une unité de régulation (94) connectée à l'unité d'exploitation (48) et servant au réglage automatique de la pression trans-membrane.

8. Dispositif selon la revendication 7, caractérisé en ce que l'unité de régulation (94) pour le réglage automatique de la pression trans-membrane, est connectée à un moteur d'entraînement (96) servant à manoeuvrer un étrangleur (92) placé sur la conduite sanguine de retour (32a, 32b).

9. Dispositif selon la revendication 7 ou 8, caractérisé en ce que l'unité de régulation (94) est connectée à un organe d'affichage (97) pour afficher et surveiller les valeurs limites de la grandeur servant au réglage de la pression trans-membrane.

10. Dispositif selon les revendications 7 et 8, caractérisé en ce que l'organe d'affichage (97) est réalisé pour afficher et surveiller les valeurs limites de la position du moteur d'entraînement (96).

11. Dispositif selon une des revendications 2 à 10 précédentes, caractérisé en ce qu'il comporte un dispositif pour l'étalonnage périodique automatique, dont fait partie une unité (70) de commande d'étalonnage agissant sur des vannes (72, 74) pour fermer l'espace (28) du dispositif (24) de dialyse ou de filtration, espace qui est destiné à être parcouru par du liquide de dialyse et/ou à l'évacuation de filtrat, avec détermination par l'unité d'exploitation de la différence qui s'établit lors de ce processus entre les signaux de mesure des deux capteurs de débit (22, 42), et que l'unité d'exploitation comporte des dispositifs pour la mémorisation de cette différence et pour la correction, en fonction de la grandeur de cette différence, du point zéro du signal de différence formé lors de la phase de fonctionnement faisant suite à l'étalonnage.

12. Dispositif selon une des revendications 2 à 11 précédentes, caractérisé en ce que les axes longitudinaux des appendices latéraux (112, 114) de l'insert de mesure (100) forment un angle de l'ordre de 10 à 60$^\circ$, de préférence de 30$^\circ$, avec l'axe longitudinal du canal d'écoulement (106) traversant l'insert de mesure.

13. Dispositif selon une des revendications 2 à 12 précédentes, caractérisé en ce que le support (102, 102a; 104, 104a) de l'insert de mesure (100) comporte des transducteurs ultrasonores, possédant des éléments transducteurs piézoélectriques (116, 118) et des pièces de couplage (120, 122) reliées à ces éléments et qui sont appliquées sous pression contre les faces de couplage (124, 126) situées aux extrémités des appendices latéraux (112, 114).

14. Dispositif selon la revendication 13, caractérisé en ce que la partie des pièces de couplage en contact avec les faces de couplage (124, 126) est faite d'un matériau doué d'élasticité caoutchoutique.

15. Dispositif selon la revendication 13, caractérisé en ce que le support pour l'insert de mesure (100) est composé d'une partie fixe (102, 102a) contenant les transducteurs ultrasonores et d'une partie (104, 104a) montée élastiquement par rapport à elle et servant à la fixation (maintien) de l'insert de mesure et à la génération de la force d'application contre les faces de couplage.

16. Dispositif selon la revendication 13, caractérisé en ce que les transducteurs ultrasonores (116, 116a) côté émission, servant à la génération des ultrasons, sont commandés par le même générateur haute fréquence (142).

FIG. 1

FIG. 2

FIG. 3

FIG. 4

FIG. 5

FIG. 6

FIG. 7